# EUROPEAN PATENT APPLICATION

(11) **EP 4 410 945 A1**
(43) Date of publication of application: **07.08.2024**
(21) Application number: 23305139.0
(22) Date of filing: 02.02.2023
(51) Int. Cl.: C12M 1/00, C12M 1/42

(54) **ELECTROPORATION DEVICE AND METHOD**

(71) Applicant: Cellectis S.A., 75013 Paris (FR)
(72) Inventor: DERLIQUE, Damien, 75013 PARIS (FR); SOURDIVE, David, 75013 PARIS (FR)
(74) Representative: Santarelli

(57) **Abstract**

A device is provided for filling and emptying of an electroporation chamber. In particular, the device comprises a tubing set (10) having a main tube (11) with a first end (12) for connection to a cell suspension input bag (1) and a second end for connection to an electroporation chamber (3). A first branch tube from a first junction (16) with the main tube has at its other end a connection for an exogenous material input bag (2). Preferably, the first junction is a T-junction and more preferably the first branch tube is connected to the stem of the T of the T-junction. A second junction between the first junction (16) and the connection to the electroporation chamber leads to a second branch tube (20) for connection to an output container (4). Further, one or more pumps are arranged to act upon the main tube, the first branch tube and the second branch tube respectively so as to displace fluid therein. During operation, the one or more pumps are controlled such that mixing of the cell suspension from the cell suspension input bag with exogenous material from the exogenous material bag occurs substantially at the first junction (16).

## Description

### Field of the invention

This invention relates to an electroporation machine. In particular, it relates to a modular electroporation system and to the method of operation of this system which are especially adapted for clinical and industrial applications.

### Background

Electroporation is understood as the use of electrical pulses to reversibly destabilize biological membranes such as vesicle or cell membranes and during the period of destabilization to provide a pathway for the introduction of exogenous material into the vesicles or cells. Typically, such introduced exogenous material can be genetic material to obtain genetically modified cells used for research or therapeutic purposes. Both the cells and the exogenous material are supplied as a liquid suspension. Of considerable interest in recent years has been the use of electroporation to introduce mRNA into cells that are readily translated into proteins that confer additional functions to those cells. Cell therapy is an emergent area of medicine that requires sterile and reliable manufacturing facilities where primary cells originating from donors or patients can be genetically modified in large batches pursuant to strict good manufacturing practices (GMP). The production of clinical batches of allogeneic CAR T-cells, for instance, which requires several gene editing steps to ablate external receptors and express artificial chimeric antigen receptors (CAR) [Depil, S., et al. (2020) 'Off-the-shelf' allogeneic CAR T cells: development and challenges. Nat Rev Drug Discov 19: 185-199] is particularly challenging in that respect.

For industrial and commercial applications, it is vital that quality and consistency of result be maintained throughout a batch of material and across multiple batches and that the batches be generated in a closed system even if several electroporation are sequentially performed. In the particular situation of primary cells, such as originating from one donor (i.e. in limited number), it is critical that the cells be preserved, which means that the electroporator system has to ensure homogeneity of the mixed cells and medium conductivity.

Where larger volume of material is involved, upscaling requires reliable systems that can be monitored from outside the system, while being cleanable with disposable electroporation chambers.

US 2006/0089674 discloses a system comprising a pulse voltage waveform generator, a switching device to connect the anode or cathode of the pulse voltage waveform generator to an electroporation chamber, and an electrode array within the electroporation chamber to convert the pulse voltage into a pulsed electric field within the chamber. Also disclosed is a cycle of filling of the electroporation chamber with a suspension of cells and exogenous material, electroporation of that mixture and emptying of the chamber in situations where the amount of cell suspension to be processed exceeds the capacity of the chamber.

In such systems, mixing of the exogenous material and the cell suspension is typically by injecting of the exogenous material into a container, typically a bag, of cell suspension prior to starting of the electroporation cycle. This however results in poor outcomes as the exogenous material is often unstable and is degraded by prolonged contact with the cell suspension prior to electroporation.

US 2011/142813 describes an electroporation device having an electroporation chamber having two inlet ports. Additionally the chambers has a vent port and an outlet port. During charging of the chamber, the cell suspension is introduced through one of the inlet ports and the liquid suspension of the exogenous material through the other inlet port. The shape of the chamber is designed to promote efficient mixing. Contact of the two liquids occurs first in the chamber thus reducing contact of these liquids prior to electroporation. This arrangement, however, suffers from the disadvantage that it is difficult to guarantee proper mixing and of the absence of air bubbles in the chamber. Such bubbles can result in destructive arcing.

So far, all these systems have not proven to be suitable for the manufacturing of genetically engineered therapeutic cells under good manufacturing practices (GMP) at the industrial scale.

It is therefore the objective of the present invention to present an electroporation system which is both inexpensive to construct and operate, and which ensures good electroporation results.

Beyond the production of therapeutic grade cells, applications can be found in various area of research or of industrial processes such as the transformation of cells lines, bacteria, yeasts, algae, plant cells, protoplasts for bioproduction, or the production of vaccines, as non-limiting examples.

### Definitions

The system of the invention is designed to process vesicles, which can be artificial or living cells. The system has been particularly designed for the electroporation of cells, such as cell lines or primary cells.

Cell means a living cell that has been cultured or placed into suspension.

By "primary cell" or "primary cells" are intended cells taken from living tissue (e.g. biopsy material) and established for growth in vitro for a limited amount of time, meaning that they can undergo a limited number of population doublings. Primary cells are opposed to continuous tumorigenic or artificially immortalized cell lines. Non-limiting examples of such cell lines are CHO-K1 cells; HEK293 cells; Caco2 cells; U2-OS cells; NIH 3T3 cells; NSO cells; SP2 cells; CHO-S cells; DG44 cells; K-562 cells, U-937 cells; MRC5 cells; IMR90 cells; Jurkat cells; HepG2 cells; HeLa cells; HT-1080 cells; HCT-116 cells; Hu-h7 cells; Huvec cells; Molt 4 cells. Primary cells are typically used in cell therapy as they are deemed more functional and less tumorigenic.

Cells differentiated from stem cells, such as cord blood stem cells, progenitor cells, bone marrow stem cells, hematopoietic stem cells (HSC) and induced pluripotent stem cells (iPS) are considered as primary cells as per the present invention.

In general, primary cells originate directly or indirectly, preferably directly, from donors or patients through a variety of methods known in the art, as for instance by leukapheresis techniques as reviewed by Schwartz J.et al. [Guidelines on the use of therapeutic apheresis in clinical practice-evidence-based approach from the Writing Committee of the American Society for Apheresis: the sixth special issue (2013) J Clin Apher. 28(3):145-284].

Particular attention has been paid to immune cells in this invention, such as T-cells and NK cells, stem cells, such as Hematopoietic Stem and Progenitor Cells, Embryonic Stem (ES) cells or induced Pluripotent Stem (iPS) cells in view of producing clinical batches of therapeutic cells for cell therapy.

By Electroporation is meant the creation of transient permeability in cell membranes using pulsed electric fields without loss of cell viability.

Exogenous material is any substance outside living cells. For the purposes of this disclosure, it is material that is to be delivered into cells. A non-exclusive list of materials is polypeptides, polynucleotides, pharmaceuticals, polymers, carbohydrates and combinations of these in the same or different molecules. Examples of polypeptides and polynucleotides are proteins, and DNA or RNA (including mRNA, RNAi) respectively. Exogenous material for use in electroporation may include combinations of the above such as Ribonucleoprotein (protein and RNA complex).

Port: An opening through which gasses or liquids may pass. The direction of flow will depend on the use of the port.

PulseAgile protocol: A sequence of at least three waveforms that has one, two, or three of the following characteristics (1) at least two of the at least three waveforms differ from each other in waveform amplitude, (2) at least two of the at least three waveforms differ from each other in waveform width, and (3) a first waveform interval for a first set of two of the at least three waveforms is different from a second waveform interval for a second set of two of the at least three waveforms. Examples of such agile pulse sequences are described in patent US6010613.

Suspension: Insoluble particles such as living cells, suspended in a water-based liquid.

### Summary of the invention

The invention relates to a device for filling and emptying of an electroporation chamber. In particular, the device comprises a tubing set having a main tube with a first end for connection to a cell suspension input bag and a second end for connection to an electroporation chamber. A first branch tube from a first junction with the main tube has at its other end a connection for an exogenous material input bag. Preferably, the first junction is a T-junction and more preferably the first branch tube is connected to the stem of the T of the T-junction. A second junction between the first junction and the connection to the electroporation chamber leads to a second branch tube for connection to an output container. Further, one or more pumps are arranged to act upon the main tube, the first branch tube and the second branch tube respectively so as to displace fluid therein. During operation, the one or more pumps are controlled such that mixing of the cell suspension from the cell suspension input bag with exogenous material from the exogenous material bag occurs substantially at the first junction.

In further arrangements, one or more bubble detectors are configured to detect the presence of gas or liquid within the tubing set. One or more valves may be positioned also act on the main tube, the first branch tube and the second branch tube respectively so as to control the flow of fluid therein.

In a further aspect, the device further comprises a third branch tube having at the one end a third junction with the main tube and having the other end being for connection with a washing solution input container wherein the third junction is positioned between the first junction and the second junction. This aspect also provides for a fourth branch tube having at the one end a fourth junction with the second branch tube and having the other end being for connection with a washing solution waste bag.

The invention also relates to an electroporation system comprising the electroporation device described above. Specifically, the system comprises the electroporation device as described above, a cell suspension bag connected to the first end of the main tube and an electroporation chamber having an entrance port connected to the second end of the main tube. The electroporation chamber further comprises a venting port, fluidly connect to a venting tube, and electroporation electrodes. The system further comprises an exogenous material input bag connected to the first branch tube and an electroporated cell suspension output bag connected to the second branch tube. Optionally a bubble detector is configured to detect the presence of fluid in the venting tube. Control circuitry is configured to supply electroporation signals to the electroporation electrodes of the electroporation chamber and to control the one or more pumps and the one or more valves based on at least signals from the one or more bubble detectors.

In yet a further aspect, the invention relates to a method of operation of the electroporation system described above. The method comprising providing a tubing set, providing an electroporation chamber, providing a stock bag containing a cell culture in liquid suspension and a stock bag containing exogenous material in liquid suspension. A predefined quantity of the cell culture and the exogenous material is introduced into the electroporation chamber and wherein the cell culture and the exogenous material are mixed within the tubing set prior to entering the electroporation chamber. Electroporation signals are supplied to the electroporation chamber so as to cause electroporation. Following this, the electroporation chamber is emptied into a storage container.

The method may further comprise a washing step in which a waste bag for receiving discarded washing solution and a stock bag of washing solution is also provided. Following emptying of the electroporation chamber, a predefined quantity of washing solution is introduced from the stock bag of washing solution into the electroporation chamber. This is subsequently discharged from the electroporation chamber into the waste bag.

In preferred arrangements, the one or more pumps of the system described above is operated at least in part based on the signals provided by the one or more bubble detectors. Further, the one or more valves operable to prevent the undesired flow of unprocessed cell suspension from the cell suspension bag and/or exogenous material suspension from the exogenous material bag; backflow of electroporation product into the stock bags; and flow of unprocessed cell suspension and exogenous material suspension into the second branch tube and hence the product bag.

In yet a further aspect, the invention encompasses the steps of introducing cells into said system, mixing the cells with an exogenous material, introducing said exogenous material into the cells by using this system for manufacturing modified cells, especially genetically modified therapeutic cells ready to be freshly used or to be frozen.

Thanks to the configuration of the electroporation device described above, effective electroporation of a cell suspension and exogenous material can be made. Specifically, the electroporation device allows the use of commercially available tubing and electroporation chambers or cuvettes allowing for cost reductions and economies of scale. The requirement for bespoke electroporation chambers is eliminated whilst also the degradation of the exogenous material caused by prolonged contact with the cell suspension prior to electroporation is minimized.

Other particularities and advantages of the invention will also emerge from the following description.

In the accompanying drawings, given by way of non-limiting examples:
Figure 1 is a schematic representation of an electroporation system according to the invention;
Figure 2 is a schematic representation of the electroporation system of figure 1 further including facility to wash the chamber after use;
Figure 2a is a schematic representation of an electroporation chamber;
Figure 3 is a schematic representation of a tubing set forming part of the system of figure 1;
Figure 4 shows a T-junction forming part of the tubing set of figure 3;
Figure 5 is a schematic representation of the electroporation device forming part of the system of figure 1;
Figure 6 is a flowchart showing a method of performing electroporation according to the system of figure 1;
Figure 7 is a flowchart showing a method of performing electroporation according to the system of figure 2;
Figure 8 is a realistic representation of the electroporation device forming part of the system of figure 2;
Figure 9 shows the percentage of TCRab KO obtained in T-cells electroporated with TRAC TALEN mRNAs after each electroporation cycle performed with the GeneEngine connect to PulseAgile electroporator;
Figure 10 shows the percentage of TCRab KO obtained in T-cells electroporated with TRAC TALEN mRNAs at the different conditions detailed in example 2. C3: condition 3, R1C1 ratio1 and first cycle; R1C2: ratio 1 and second cycle; R2C1: ratio 2 and first cycle; R2C2: ratio 2 and second cycle.

### Detailed Description

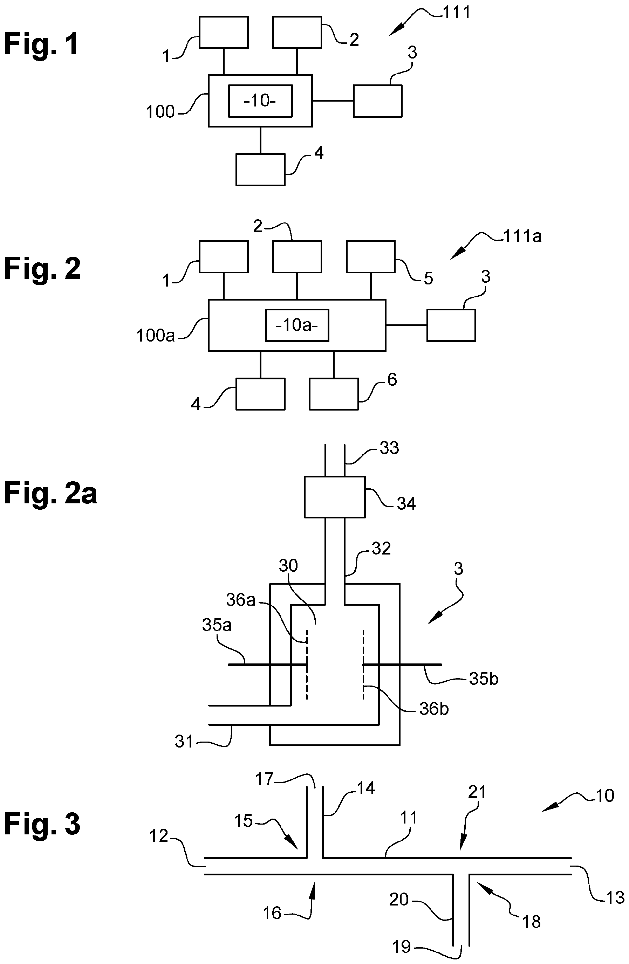

Fig. 1 is a schematic representation of an electroporation system comprising an electroporation device 100, a container 1 for holding a suspension of cells to be treated by electroporation, a container 2 for holding a suspension of the exogenous material to be inserted into the cells, an electroporation chamber 3 and an output container 4 for collecting the product of the electroporation process. Typically, such containers are flexible bags formed of for example plastic. For the present disclosure, these containers are referred to as bags. This should not be regarded as limiting.

The cell suspension bag 1 and the exogenous material bag 2 are fluidly connected to the electroporation device 100. Collectively, the cell suspension bag 1 and the exogenous material bag 2 are referred to as the supply bags 1, 2. The electroporation device operates to draw a predetermined quantity of the suspension of cells from the cell suspension bag 1 and a predetermined quantity of the suspension of exogenous material form the exogenous material bag 2, to mix these suspensions prior to delivery to the electroporation chamber 3.

In some arrangements a mixing device is configured for agitating the cell suspension bag 1 and its contents. The mixing device may oscillatingly compress the bag so as to cause the flow of liquid within the bag. Such flow ensures that the concentration of cells within the bag is maintained uniform and prevents cells from settling within the bag so as to form a concentration gradient across the bag. Cells are also thereby prevented from "sticking" to the walls of the bag. A uniform concentration within the bag ensures a non-changing concentration of cells entering the electroporation device as the bag is emptied. This is important for ensuring uniform and reproducible electroporation outcomes.

The system of fig 1 further comprises a signal generator configured to generate electroporation signals. In preferred arrangements, the signal generator is configured to produce signals conforming to the PulseAgile protocol. The signal generator is electrically connected to electrodes within the electroporation chamber configured to conduct the electroporation signals to the content of the electroporation chamber.

The output bag 4 for collecting the product of the electroporation process is fluidly connected to the electroporation device 100 and is configured to receive product from the electroporation chamber 3 following electroporation.

In addition to the features of the electroporation system 111 depicted in figure 1, the electroporation system 111a of fig. 2 comprises a bag for containing a washing solution 5 fluidly connected to an electroporation device 100a. The electroporation device 100a is configured such that the washing solution is directed to the electroporation chamber 3 following electroporation and discharge of electroporation product. Cleansing occurs by filling and discharging the electroporation chamber 3 with washing solution. A washing solution waste bag 6 is provided and is fluidly connected to the electroporation device for receiving waste washing solution.

Fig. 2a shows, in schematic form, the electroporation chamber 3 in greater detail. The electroporation chamber comprises an active chamber 30 having a predetermined volume, an entrance port 31 and a venting port 32. Within the active chamber 30 are situated electroporation electrodes 35a, 35b to which electroporation signals can be applied to material within the active chamber 30. The electroporation electrodes are electrically connected to electrical leads 36a, 36b respectively for connection to the signal generator.

Now will be described in more detail the electroporation device 100. A principal component of the electroporation device 100 is the tubing set 10 illustrated in fig. 3. The tubing set comprises a main tube 11 having a first end 12 for connection to the cell culture input bag 1 and a second end 13 for connection to the electroporation chamber 3. Also forming part of the tubing set 10 are first and second branch tubes 14, 20. The first branch tube 14 has at the one end 15 a first junction 16 with the main tube 11, the other end 17 being configured for connection with the exogenous material bag 2. The second branch tube 20 has at the one end 18 a second junction 21 with the main tube 11, the other end 19 being configured for connection to the output bag 4. The second junction 21 is positioned between the first junction 16 and the second end 13.

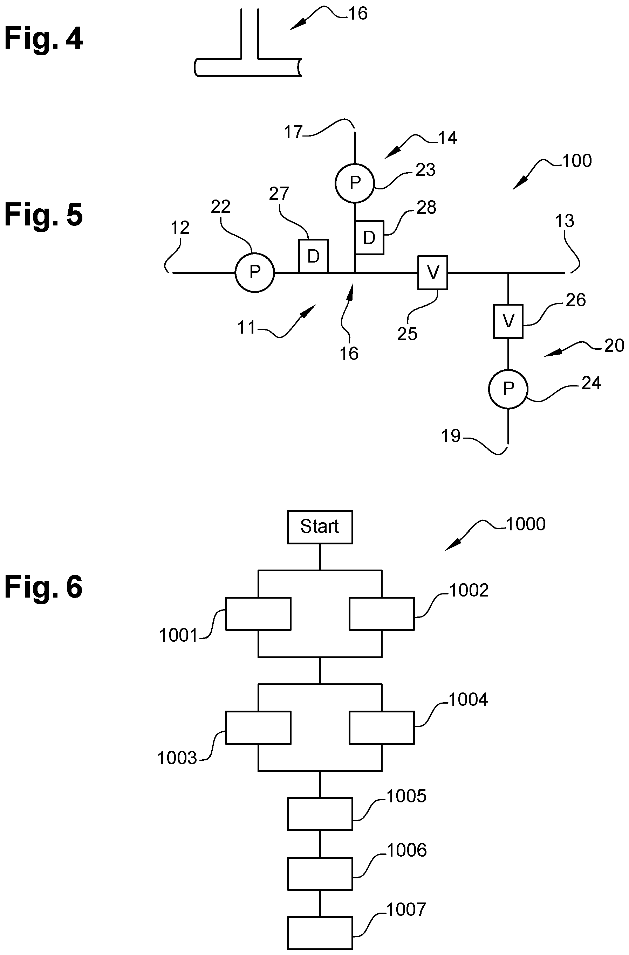

Shown in fig. 4 is a preferred form of the first junction 16 between the main tube 11 and the first branch tube 14. As illustrated, the first junction 16 takes the form of a "T" wherein the bar of the T forms the path of the main tube 11 and the stem of the T, the first branch tube. The inventors have found efficient mixing of the cell suspension and exogenous material suspension to occur at such junctions. Other configurations are also possible.

In order to move liquid to and from the electroporation chamber 3 and to provide a defined volume and an adequate quantity of cells and exogenous material a number of pumps are used. Such pumps are typically peristaltic pumps configured to pump the contents of the tubing set 10 without coming into direct contact with the liquids within.

The pumps are electrically connected to control circuitry configured to control operation of the pumps and thus the movement of fluids within the tubing set 10.

Fig 5 shows an arrangement of pumps placed on the tubing set 10 described above. A first pump 22 is arranged on the main tube 11 at a location between the first end 12 and the first junction 16. Operation of the first pump 22, during operation of the electroporation device, causes the contents of the cell suspension bag 1 to be pumped into the electroporation chamber 3. A second pump 23 is arranged on the first branch tube 14. Operation of the second pump 23 causes the contents of the exogenous material suspension bag 2 to be pumped into the electroporation chamber 3. Operation of the first pump 22 and the second pump 23 simultaneously causes mixing of the cell suspension and exogenous material suspension to occur at the first junction. A third pump 24 is arranged on the second branch tube 20. Operation of the third pump 24 causes the contents of the electroporation chamber 3 to be pumped towards the output bag 4. The first 22, second 23 and third 24 pumps are electrically connected to the control circuitry.

In order to prevent the movement of liquid within the tubing set, a number of valves are employed. Such valves are typically pinch valves operable to prevent flow in the tubing set 10 without coming into direct contact with the liquids within. Fig. 5 shows an arrangement of valves on the tubing set 10 of the electroporation device 100. A first valve 25 is arranged on the main tube 11 at a location between the first junction 16 and the second junction 21. When operated, the first valve 25 serves to restrict or prevent the flow of liquid from the electroporation chamber 3 to the supply bags 1, 2 or from the supply bags 1, 2 to the electroporation chamber. It also avoids further mixing of cells and exogenous material allowing to have a precise ratio of cells and exogenous material that will enter into the electroporation chamber. A second valve 26 is arranged on the second branch tube 20 at a location between the second junction 21 and the third pump 24. When operated, the second valve 26 serves to restrict or prevent the flow of liquid from the main tube 11, that is to say unelectroporated cells with exogenous material, to the second branch tube 20 and thus to the output bag 4.

The first valve 25 and the second valve 26 are electrically connected to and configured for control by the control circuitry.

As discussed in the introduction above, the presence of bubbles within the electroporation chamber 3, is highly injurious to the electroporation process. In addition, to have a precise control of the liquid flow and to detect the end of the electroporation process bubble detectors are placed at different location on the tubing sets. As shown in fig. 5, a first bubble detector 27 is placed on the main tube 11 at a location between the first pump 22 and the first junction 16. A second bubble detector 28 is placed on the first branch tube 14 at a location between the second pump 23 and the first junction 16.

The first bubble detector 27 and the second bubble detector 28 are electrically connected to the control circuitry causing the presence of bubbles in the tubing to be registered by the control circuitry.

With reference again to fig. 2a, the venting port 32 is fluidly connected to a venting tube 33 through a third bubble detector 34. The third bubble detector is electrically connected to the control circuitry.

Referring again to the electroporation system 111a shown in fig 2, this system comprises the washing solution bag 5 and the washing solution waste bag 6 in addition to the features described above in relation to the system 111 depicted in fig. 1.

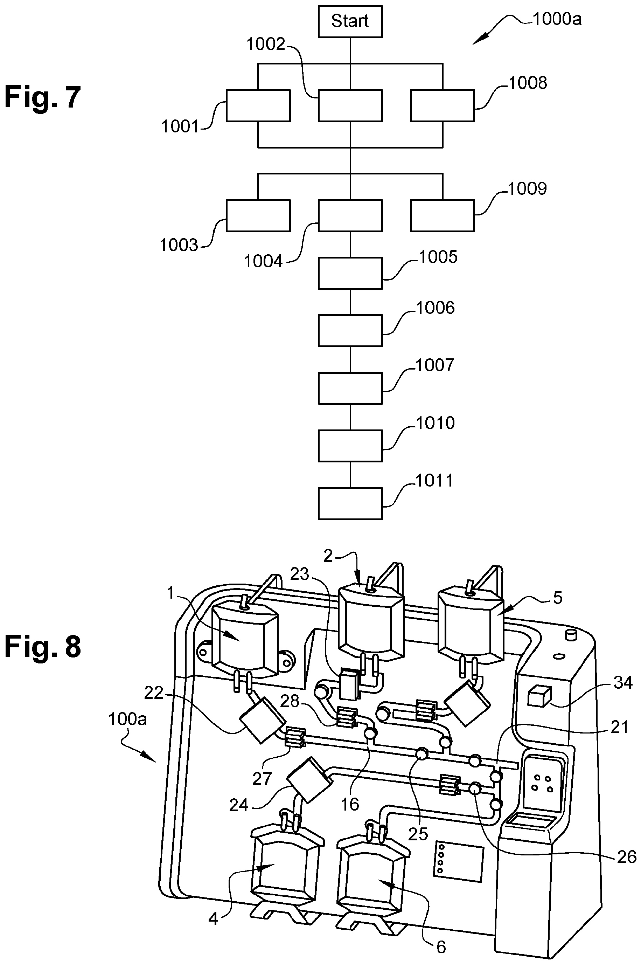

Figure 8 is a portrayal of the electroporation device 100a of the electroporation system 111a. The embodiment shown is the GeneEngine^{™} machine. Also shown in fig. 8 is the third bubble detector 34 which is configured to be placed on the venting tube 33 of the electroporation chamber 3 when this is connected to the electroporation device 100a. The tubing set comprises the following features in addition to the tubing set 10 depicted in fig. 3. A third branch tube is connected to the main tube at a third junction located on the main tube between the first junction 16 and the second junction 21. The third branch tube is configured at its other end of connection to the washing solution bag 5. A fourth branch tube is connected to a junction with the second branch tube at one end and configured for connection to the washing solution waste bag 6 at the other.

A fourth pump is arranged on the third branch tube. Also arranged on the third branch tube are a third valve and a fourth bubble detector. A fifth bubble detector may be placed on the second branch tube. Additional valves can be arranged as required to prevent the flow of liquid during the operation of the first to fourth pump into tubing where it is not desired. Each of the pumps, and bubble detectors is respectively electrically connected to the control circuitry.

It will now be described a method 1000 of operating the system 111 described above and depicted in fig. 1. The flowchart of fig. 6 depicts this method. The method 1000 comprises providing 1001 the tubing set 10 and providing 1002 the electroporation chamber. Further, are provided the consumables being 1003 the stock bag 1 containing a cell culture in liquid suspension and 1004 the stock bag 2 containing exogenous material in liquid suspension. The tubing set is, by operation of pumps and valves, acted upon such as to introduce 1005 to the electroporation chamber 3, a predefined quantity of the cell culture from the cell culture bag 1 and a predefined quantity of the exogenous material from the exogenous material bag 2 into the electroporation chamber 3.

By operating the first pump 22, a predefined quantity of cell suspension is introduced into the electroporation chamber 3. By operating the second pump 23, exogenous material suspension is introduced into the electroporation chamber 3. It is a feature of the present method 1000 that the cell culture suspension and the exogenous material suspension are mixed within the tubing set. More specifically, mixing occurs at the junction 16 between the main tube 11 and the first branch tube 14. This can be achieved by operating the first pump 22 and the second pump simultaneously.

During operation of the first 22 and second 23 pump, the second valve 26 is held in a closed state so as to prevent liquid from flowing into the second branch tube 20 and into the output bag 4.

Once the mixture of cell culture suspension and the exogenous material suspension has been introduced to the electroporation chamber, an electrical current consisting of electroporation signals are supplied 1006 to the electroporation electrodes 35a, 35b of the electroporation chamber. Preferably, the electroporation signals conform to the PulseAgile protocol as defined above. As explained above, the electroporation chamber comprises electrodes for supplying an electric field to the contents. Upon supplying of the electroporation signals, the exogenous material is incorporated into the cells in the manner described in the introduction above.

Following the electroporation step 1006, the electroporated contents of the electroporation chamber is emptied 1007 into the output bag 4. For the system depicted in fig. 1, the emptying step 1007 is performed by having the first valve in a closed state, the second valve 26 in an open state and operating the third pump 24.

The steps of introducing 1005, supplying 1006 and emptying 1007 can be repeated as desired or until the consumables have been exhausted.

Fig. 6 shows a method 1000a for operating the device 100a and system 111a depicted in figs. 2 and 8. System 111a differs from system 111 with the addition of a washing facility. The corresponding method comprises the following additional steps. At step 1008, a bag 5 containing a washing solution is provided. A waste bag 6 for receiving waste washing solution is provided 1009.

Following emptying of the electroporation chamber 3, the following additional steps can be performed to cleanse the electroporation chamber. A predefined quantity of a washing solution is introduced 1010 from the washing solution bag to the electroporation chamber. A predefined sitting time having elapsed the washing solution is discharged 1011 from the electroporation chamber to the waste bag 6. Cleaning is performed at the end of a batch of electroporation cycles and as required.

Control of the supply, by the signal generator, of electroporation signals to the electroporation electrodes 35a, 35b of the electroporation chamber 3 as well as control of the one or more pumps 22, 23, 24 and of the one or more valves 25, 26 is by the control circuitry. The control circuitry is configured to implement the method 1000, 1000a described above. The control circuitry is configured such that control of the one or more pumps 22, 23, 24 and of the one or more valves 25, 26 is based in part on signals received from the one or more bubble detectors 27, 28 and/or the third bubble detector 34. The presence of bubbles in the tubing set is indicative of bubbles in the electroporation chamber 3. Detection of fluid by the third bubble detector may indicate a misfunction of the pumping sequence. As described above, bubbles in the electroporation chamber 3 is highly detrimental. For example, the control circuitry is configured such that if bubbles are detected by either the first bubble detector 27 or the second bubble detector 28, indicating the presence of bubble in the cell culture suspension and/or the exogenous material suspension respectively, the electroporation process is terminated and a signal transmitted to the user.

While the electroporation system described herein can be used for any type of cells, especially for bioproduction or research purposes, such as the screening of genetically engineered cells, it has been particularly designed for the manufacturing of therapeutic cells. Thus, an aspect of the present invention is the use of such system for introducing an exogenous material into a cell and obtaining the subsequent genetic modification of said cell by said exogenous material.

By exogenous material is encompassed a polypeptide or polynucleotide that can be expressed into the cell or any molecule that will have an effect on genome expression, such as introducing a mutation. This exogenous material can be for instance a transgene, a mRNA encoding a protein, vectors comprising an expression cassette, single strand DNAs or DNA templates for insertion into the genome, RNAi, transcription, Ribonucleoprotein (RNP, such as a Cas nuclease along with a guide RNA) or translation inhibitors. For the purpose of engineering therapeutic immune cells, such as T-cells or NK-cells expressing chimeric antigen receptors (CARs) or recombinant TCRs, the exogenous material can be a rare-cutting endonuclease or a base editor, or a polynucleotide encoding thereof, that can specifically cleave or introduce a mutation into a genomic target sequence [Bailey, S.R., Maus, M.V. (2019) Gene editing for immune cell therapies. Nat Biotechnol 37, 1425-1434]. By exogenous genetic material is meant a polynucleotide encoding a protein that can be transiently expressed in such cells or designed to be integrated into their genome. According to some aspects, the exogenous material can be a mutagenic molecule such as base analogs (ex: 5 bromo uracil and 2 amino purine) or intercalating agents (ex: ethidium bromide, proflavine, daunorubicin..).

Also the present invention encompasses methods of genetic modification of cells, preferably primary immune cells, wherein cells are electroporated into the electroporation system described herein and subsequently recovered from this system.

Such methods can comprise one or several of the following steps:
- Cells are provided, usually from a pre-culture, and introduced into the system as previously described;
- Cells are mixed with the exogenous material, for instance mRNA encoding a target sequence specific reagent such as a rare-cutting endonuclease or a base editor and electroporated within said system as previously described and shown in the examples;
- Cells are recovered outside the system and cultured;
- Cells can be purified and ultimately frozen, dehydrated, processed, freshly used or incorporated into compositions for therapeutic or non-therapeutic purposes.

More specifically, the present system can be implemented for producing batches of primary immune cells, preferably allogeneic CAR T-cells, wherein immune cells are provided from donors, patients or derived from stem cells. Such cells are mixed within the tubing set with polynucleotides or ribonucleoprotein encoding a rare cutting endonuclease or a base editor targeting T-cell receptor (TCR) in said cells. The mixture, free of bubbles, is then pushed to the electroporation chamber for electroporation. Several successive cycles can be performed. The electroporated cells are then recovered and cultured. Upon expression of the rare-cutting endonuclease or base editor targeting the TCR gene, the cells remaining TCR positive are removed from the culture of engineered cells. The resulting TCR negative cells can be transduced with viral vectors to arm them with transgenic CARs or TCRs to confer them a specificity toward cancer markers, such as CD19, CD22, CD20, CS1, BCMA, CD123, ROR1, GD2, CD33, CD38, CD70, FAP, IL3RA, MUC1, Mesothelin, PSCA, Claudin 18.2 and EGFRvIII as non-limiting examples, to produce one or several batches of CAR-T cells therapeutic compositions for preclinical or clinical applications.

The electroporation system according to the invention allows successive cycles of electroporation without opening the system in view of producing unique batches of engineered vesicles or cells. In some embodiments, the above methods of the invention can comprise more than 5 cycles, preferably more than 10, more preferably more than 15, 20, 25, 30, 35 and even more than 40 cycles and up to 45 cycles. Each cycle can electroporate between 5 and 20 ml, preferably about 10 ml, especially for T-cells, NK cells or HSCs, which can amount more than 10⁹ electroporated cells or vesicles. In turn, such system can process more than 10¹⁰ cells when successive cycles are applied, in order to produce batches that can amount up to 5.10¹⁰ engineered cells when more than 30 cycles are performed.

### Examples

There follow examples demonstrating advantageous results achieved using the system according to the present invention. By way of comparison, Example 1 demonstrates the detrimental effect of prolonged contact between cells and exogenous material. In Example 2, the advantageous results obtained from the electroporation system of the present invention are demonstrated.

### Example 1: GeneEngine using mRNA and T-cells mix

PBMCs were thawed on day 0, activated on day 1 with 3.8 mL TransACT 5X for 64 hours and for 3.17 10⁸ CD3⁺ cells. Cells were then seeded in two GRex500 on day 4 for a 10 days expansion period before electroporation in Complete Medium (OpTmizer^{™} CTS^{™} T-Cell Expansion Basal Medium, Gibco + 5% Human Serum AB).

After 10 days of expansion, T-cells were resuspended at 10⁸ cells/ml in Buffer T (Harvard Apparatus). The mRNAs encoding TALEN targeting TRAC locus were prepared in Buffer T at 1 mg/mL. Electroporation was performed using the GeneEngine^{™} machine connected to PulseAgile electroporator (updated to drive GeneEngine^{™}) with T-cells already mixed with mRNAs at 0,5 µg/10⁶ cells per TALEN arm. Each electroporation cycle last for one minute. After each electroporation cycle, electroporated T-cells were recovered from the output bag, seeded in complete medium at 1.9 10⁶ cells/mL, incubated for 18 hours at 30°C. T-cells were then transferred in pre-warmed complete medium and incubated at 37°C for 3 days. TCRαβ knock out was then measured by flow cytometry using antibodies as described in Poirot et al. Cancer Research 2015.

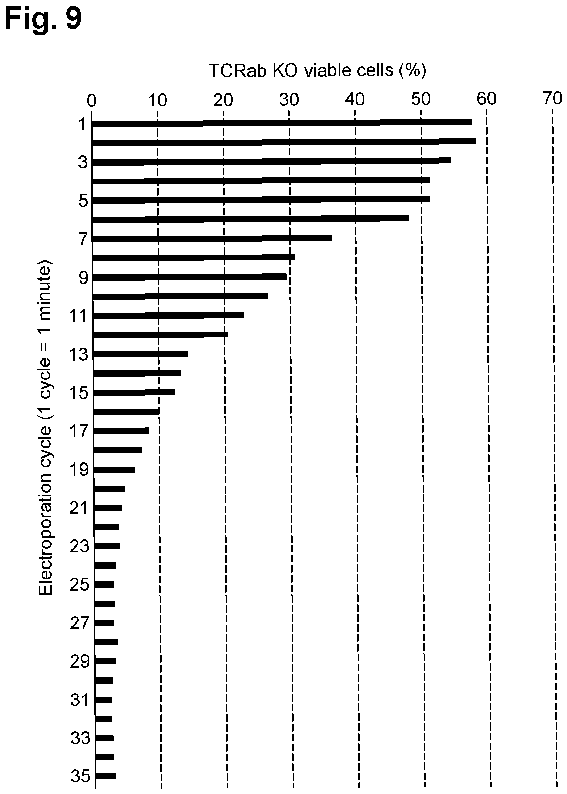

Figure 9 shows the percentage of TCRαβ knock out obtained in T-cells electroporated with TRAC TALEN mRNAs after each electroporation cycle performed with the GeneEngine connect to PulseAgile electroporator.

Results in Figure 9 show that TCRαβ knockout efficacy, (TCRab KO viable cell) was decreasing as the number of electroporation cycle (and therefore time) was increasing, demonstrating that a prolonged contact of mRNA with T-cells lowered the TCRαβ knockout efficacy. The number of cycles shown in figure 9 is 35 cycles. This strongly suggest that mRNAs are degraded in presence of T-cells. Therefore, if several numbers of electroporation are needed, T-cells and mRNA should not be in contact for longer than necessary prior to electroporation.

### Example 2: GeneEngine with mRNA and T cells separated and mixed just prior electroporation in a T tubing set according to the invention.

Jurkat cells were cultivated in Complete Medium (OpTmizer^{™} CTS^{™} T-Cell Expansion Basal Medium with 10% FBS (Fetal Bovine Serum), Glutamax (1% v/v, Gibco), CTS^{™} OpTmizer^{™} T-Cell Expansion Supplement (26 mL / liter of medium) and Penicillin/Streptomycin solution (100 U/mL, Gibco) in flasks until reaching at least 10⁹ cells. Cells were then re-suspended in 1L Complete Medium at 10⁶ cells / mL in a CellBag installation (Xuri) and cultivated with rocking (8 rpm, 6° inclination) in 5% CO₂, 21% O₂, gas inlet at 0.2 mL/min and at 37°C. After 10 days of expansion, either T-cells and TRAC TALEN arms mRNAs were re-suspended at 105,3 10⁶ cells/ml and 1 mg/mL in Buffer T, respectively (see below ratio 1, R1); or T-cells and TRAC TALEN arms mRNAs were re-suspended at 125 10⁶ cells/ml and at 1 mg/mL in Buffer T, respectively (see below ratio 2: R2).

Several conditions were then tested using GeneEngine machine connected to PulseAgile electroporator (updated to drive GeneEngine). Cells were electroporated without mRNA, (negative control). Cells and mRNA were mixed manually and directly electroporated (Positive Control 1), Jurkat cells and mRNA were left separately untouched for 30 min then mix manually and directly electroporated (Positive Control 2). Jurkat cells and mRNA were mixed manually and incubated for 30 min before electroporation (condition3: C3). Jurkat cells and mRNA were place in separated bags in the GeneEngine and a first cycle was performed: mixing the cells and mRNA in the T tubing set and directly electroporated (C1). A second electroporation cycle was performed but 30 minutes after the first cycle (C2).

Two different T-cells:mRNA volume ratios were tested: either 9,5:0,5 (corresponding to 9,5 mL of Jurkat cells and 0,5 mL of mRNA) (R1) or 8:2 (R2) but in each case these ratio led to the electroporation of the same quantity of Jurkat cells (10⁹ cells) with the same quantity of mRNA (0,5 µg/10⁶ cells per TALEN arm).

After each cycle cells were collected from the output bag, seeded in Complete Medium at 1.6 10⁶ cells/mL, incubated for 18 hours at 30°C. Cells were then transferred in rewarmed complete medium and incubated at 37°C for 3 days. TCRαβ knock out was then measured by flow cytometry using antibodies as described in Poirot et al. Cancer Research 2015.

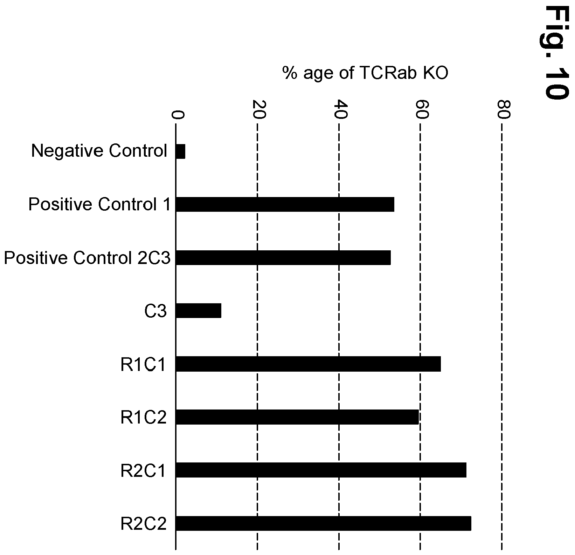

Figure 10 show the percentage of TCRαβ knock out (TCRab KO) obtained in T-cells electroporated with TRAC TALEN mRNAs at the different conditions detailed above. C3: condition 3, R1C1 ratio1 and first cycle; R1C2: ratio 1 and second cycle; R2C1: ratio 2 and first cycle; R2C2: ratio 2 and second cycle.

Results in 10; showed that TCRαβ knock out efficacy was around 50% when the cells were mixed manually just prior electroporation (Positive Control 1 and Positive Control 2). But when the cells and mRNA were mixed 30 min prior electroporation (C3), TCRαβ knockout efficacy dropped to only 10% confirming previous observations as demonstrated in Example 1 above. When mixing Jurkat cells and mRNA in the T tubing set of the GeneEngine apparatus the TCRαβ knockout efficacy was even higher than positive controls reaching between 60 to 70% efficacy whatever the volume ratio used (R1 or R2) and whenever the electroporation occurred (at the first or at the second cycle C1 or C2). These results demonstrate that mixing cells and mRNA in a T tubing set, within the GeneEngine, is well suited for efficient and successive electroporation.

The reader will appreciate the above described electroporation device, system and method of operation represents a welcome improvement in the cell industry. The disclosed device system and method allows the use of commercial available components to obtain better quality electroporation product than has until now been possible. The use of commercially available components allows for cost savings and economies of scale.

## Claims

1. A device (100) for supplying an electroporation chamber (3) comprising:
a tubing set (10) comprising:
a main tube (11) having a first end (12) for connection to a cell suspension input bag (1) and a second end (13) for connection to an electroporation chamber (3);
a first branch tube (14) having at the one end (15) a first junction (16) with the main tube (11) the other end being (17) for connection with an exogenous material input bag (2);
a second branch tube (20) having at the one end (18) a second junction (21) with the main tube (11) placed between the first junction and the second end (13), the other end (19) being for connection to an output container (4);
one or more pumps (22, 23, 24) configured to act upon the tubing set (10) and controllable to displace fluid within the tubing set (10);
**characterized in that** during operation, mixing of a cell suspension from the cell suspension input bag (1) with exogenous material from the exogenous material bag (2) occurs at the first junction (16).

2. The device (100) of claim 1 further comprising one or more bubble detectors (27, 28) configured to detect the present of gas within the tubing set (10).

3. The device (100) of claim 1 wherein the first junction (16) is a T-junction and wherein preferably, the first branch tube (14) is connected to the stemof the T of the T-junction.

4. The device (100) of claims 1 to 3 further comprising one or more valves (25, 26) acting on the tubing (10) configured to block the flow of fluid.

5. The device (100) of claim 1 to 4 wherein the one or more pumps comprise:
a first pump (22) acting on the tubing set at a location between the first end (12) of the main tube (11) and the first junction (16); and
a second pump (23) acting on the first branch tube (14).

6. The device (100) of claim 5 wherein the one or more pumps further comprises a third pump (24) acting on the second branch tube (20).

7. The device (100) of any of claims 4 to 6 wherein the one or more valves comprised a first valve (25) situated to act on the tubing between the first junction (16) and the second junction (21) and a second valve (26) situated to act on the second branch (20).

8. The device (100a) of claims 1 to 7 further comprising:
a third branch tube having at the one end a third junction with the main tube (11) and having the other end being for connection with a washing solution input container (5) wherein the third junction is positioned between the first junction (16) and the second junction (21);
a fourth branch tube having at the one end a fourth junction with the second branch tube (20) and having the other end being for connection with a washing solution waste bag (6).

9. An electroporation system (111) comprising:
the device (100, 100a) of any of claims 4 to 8;
a cell suspension bag (1) connected to first end (12) of the main tube (11);
an electroporation chamber (3) having:
an entrance port 31 connected to the second end (13) of the main tube (11);
a venting port (32) fluidly connect to a venting tube (33);
electroporation electrodes (35a, 35b);
an exogenous material input bag (2) connected to the first branch tube (14);
an electroporated cell suspension output bag (4) connected to the second branch tube (20);
optionally a third bubble detector (34) configured to detect the presence of fluid in the venting tube (33)
control circuitry configured to:
supply electroporation signals to the electroporation electrodes 36a, 36b of the electroporation chamber (3); and
control the one or more pumps (22, 23, 24) and the one or more valves (25, 26) based on at least signals from the one or more bubble detectors (27, 28).

10. A method (1000) for electroporation comprising:
providing (1001) a tubing set (11);
providing (1002) an electroporation chamber (3);
providing (1003) a stock bag (1) containing a cell culture in liquid suspension;
providing (1004) a stock bag (2) containing exogenous material in liquid suspension;
introducing (1005) a predefined quantity of the cell culture; and the exogenous material into the electroporation chamber (3) wherein the cell culture and the exogenous material are mixed within the tubing set (11) prior to entering the electroporation chamber;
supplying (1006) electroporation signals to the electroporation chamber (3) so as to cause electroporation to the contents;
emptying (1007) the electroporation chamber (3) into a storage container (4).

11. The method (1000a) of claim 10 further comprising:
providing a waste bag (6) for receiving discarded washing solution (1008);
providing a stock bag (5) of washing solution (1009);
following emptying (1007) of the electroporation chamber, introducing (1010) a predefined quantity of washing solution from the stock bag (5) of washing solution to the electroporation chamber (3);
discharging (1011) the electroporation chamber into the waste bag (6) for receiving discarded washing solution.

12. The method of claims 10 or 11 further comprising:
providing one or more pumps (22, 23, 24) configured to pump fluid within the tubing set (11);
providing one or more bubble detectors (27, 28, 34) configured to provide signals according to the presence of fluid and/or gas bubbles within the tubing set (11);
operating the one or more pumps (22, 23, 24) to cause the introducing into and/or emptying of the electroporation chamber (3);
wherein the one or more pumps (22, 23, 24) is operated at least in part based on the signals provided by the one or more bubble detectors (27, 28, 34).

13. The method of claim 10 to 12 further comprising:
providing one or more valves (25, 26) operable to prevent the flow of fluid within the tubing set (11);
operating the one or more valves to prevent:
undesired flow of unprocessed cell suspension from the cell suspension bag (1) and/or exogenous material suspension from the exogenous material bag (2) backflow of electroporation product into the stock bags (1, 2); and
flow of unprocessed cell suspension and exogenous material suspension into the second branch tube (20) and the product bag (4).

14. Application of the method of any of claims 10 to 13 to manufacture therapeutic cells for cell therapy.

15. The application of claim 14 where the therapeutic cells are immune cells in particular, such as T-cells and NK cells, or the therapeutic cells are stem cells such as Hematopoietic Stem and Progenitor Cells, Embryonic Stem (ES) cells or induced Pluripotent Stem (iPS) cells.
